# EUROPÄISCHE PATENTSCHRIFT

(11) **EP 3 490 664 B1**
(45) Veröffentlichungstag und Bekanntmachung des Hinweises auf die Patenterteilung: **09.09.2020**
(21) Anmeldenummer: 17740741.8
(22) Anmeldetag: 18.07.2017
(51) Int. Cl.: A61N 1/36, A61F 2/72, A61F 5/01, A61N 1/04, A61F 2/68, A61B 5/00, A61B 5/11, A63B 23/04, A61F 2/60, A61F 2/76, A61F 2/50, A61F 2/70, A61F 2/64, A61H 1/02, A61H 3/00

(54) **ORTHOPÄDIETECHNISCHES SYSTEM**
ORTHOPAEDIC SYSTEM
APPAREIL ORTHOPÉDIQUE

(30) Priorität: 29.07.2016 DE 102016114075
(43) Veröffentlichungstag der Anmeldung: 05.06.2019
(60) Teilanmeldung: 20187233.0
(73) Patentinhaber: Otto Bock Healthcare Products GmbH, 1110 Wien (AT)
(72) Erfinder: DIETL, Hans, 3004 Ried am Riederberg (AT); AUBERGER, Roland, 1140 Wien (AT); HOFER, Christian, 1150 Wien (AT); RUSSOLD, Michael Friedrich, 1210 Wien (AT)
(74) Vertreter: Gramm, Lins & Partner Patent- und Rechtsanwälte PartGmbB
(86) Internationale Anmeldenummer: PCT/EP2017/068101
(87) Internationale Veröffentlichungsnummer: WO 2018/019654

(56) Entgegenhaltungen:
- WO-A2-02/060311
- DE-A1-102008 011 977
- DE-A1-102014 117 663
- US-A- 5 476 441
- US-A1- 2003 093 021
- US-A1- 2004 172 097

## Beschreibung

Die Erfindung betrifft ein orthopädietechnisches System mit zumindest einer Stimulationselektrode zur Aktivierung zumindest eines Muskels und einer orthetischen oder prothetischen Einrichtung, die an einem Körper eines Patienten anlegbar und daran festlegbar ist, mit zumindest einem Gelenk, über das eine proximale und distale Komponente der orthetischen oder prothetischen Einrichtung schwenkbar miteinander verbunden sind. Die Erfindung findet Anwendung in einem Verfahren zur Steuerung eines solchen orthopädietechnischen Systems, bei dem über die zumindest eine Stimulationselektrode zumindest ein Muskel des Patienten aktiviert wird.

Aus der US 7,403,821 B2 ist ein Verfahren zur Erzeugung einer Dorsalflexion eines Patientenfußes bekannt, bei dem Stimulationselektroden und Signalerfassungselektroden an zumindest einem peripheren Nerv des Oberschenkels angeordnet wird. Über die Elektroden werden Neurosignale aufgenommen und verarbeitet, um eine Bewegung oder eine Aktion zu bestimmen, die den Zeitpunkt eines Fersenstoßes oder eines Abhebens der Ferse während des Gehens anzeigt. In Abhängigkeit von der detektierten Aktion wird die Stimulationselektrode aktiviert, um eine Dorsalflexion des Fußes des Patienten zu erzeugen.

Die EP 1 257 318 B1 betrifft eine Vorrichtung zum Erzeugen einer Dorsalflexion und zur Stimulierung einer motorischen Nervenfaser mit Mitteln zum Empfangen und Verarbeiten der detektierten Nervensignale und zum Erzeugen von Stimulationssignalen, wobei die Vorrichtung eine kombinierte Detektions- und Stimulationselektrodenvorrichtung umfasst und als eine kombinierte Elektrode ausgebildet ist. Es sind Mittel vorgesehen, um jede kombinierte Elektrode zwischen einem Detektionszustand und einem Stimulationszustand umzuschalten. Die kombinierte Detektions- und Stimulationselektrodenvorrichtung ist implantierbar ausgebildet.

Weiterhin ist aus dem Stand der Technik bekannt, eine prothetische Einrichtung, beispielsweise für eine untere Extremität, mit einer Widerstandseinrichtung auszustatten und in Abhängigkeit von ermittelten Sensorwerten eine Anpassung der Bewegungswiderstände vorzunehmen. Eine solche Vorrichtung und ein solches Verfahren sind beispielsweise aus der DE 10 2009 052 887 B1 bekannt.

Menschen, die prothetische Einrichtungen benötigen, können zusätzliche Beeinträchtigungen in der willkürlichen Aktivierung der Muskulatur aufweisen. Gleiches gilt für Menschen, die auf eine orthetische Versorgung der unteren Extremitäten angewiesen sind, beispielsweise aufgrund von Unfällen, neuronalen Schädigungen oder anderen Beeinträchtigungen der willkürlichen Aktivierung. Darüber hinaus können fehlende sensorische Rückmeldungen die Mobilität der Patienten beeinträchtigen, so dass sich ein verschlechtertes Gangbild oder auch eine grundsätzliche Beeinträchtigung der Allgemeinfunktion der unteren Extremität einstellen kann. Die Ursache dieser Beeinträchtigung können Lähmungen, Muskeldefekte nach medizinischen Eingriffen oder onkologischen Erkrankungen, Schlaganfälle, andere zerebrale Störungen oder ähnliches sein.

Darüber hinaus existieren prothetische oder orthetische Einrichtungen mit externen, häufig elektromotorischen Antrieben, um die motorischen Defekte der Muskulatur auszugleichen. Solche aktiven Prothesen oder Orthesen sind sehr schwer, benötigen einen hohen Energieaufwand und verursachen in der Regel störende Geräusche. Ein Beispiel für eine aktive Prothese ist in der US 5 246 465 A beschrieben.

Die DE 10 2014 117 663 A1 betrifft eine Einrichtung, die zur elektrischen Muskelstimulation von am physiologischen Gangbild eines Menschen beteiligten Muskeln. Die Einrichtung weist ein Element zur elektrischen Muskelstimulation auf. Ein Sensormittel zum Erfassen von für die Muskelstimulation relevanten Informationen ist mit einer Steuerung- und/oder Regelungseinheit zum Auslösen der Muskelstimulation auf Basis der durch das Sensormittel erfassten Informationen gekoppelt. Ein einziges Gyroskop dient zum Erfassen der Winkelgeschwindigkeit und der Schwungrichtung des Unterschenkels in Extensionsrichtung und Flexionsrichtung. Auf der Grundlage der Sensorinformationen errechnet eine Auswerteeinheit die Schaltzeitpunkte für die Muskelstimulation.

Die US 5 576 441 A betrifft eine Orthese mit einem Stimulator zur Stimulation von Muskeln eines Körperteils, um das Körperteil zu bewegen. Die Orthese weist ein Oberteil und ein gelenkig daran befestigtes Unterteil auf. Eine Bremse ist mit dem Gelenk gekoppelt, um den Muskel darin zu unterstützen, eine gesteuerte Bewegung der Gliedmaße auszuführen. Die Bremse weist einen veränderlichen Widerstand auf. Ein Sensor ist mit dem Gelenk gekoppelt, um ein Signal über die Drehstellung des Gelenkes bereitzustellen. Ein Computer ist mit dem Sensor gekoppelt und steuert die Bremse dergestalt, dass ein gewünschter Bewegungsverlauf ausgeführt wird. Die US 2004/172097 A1 betrifft eine medizinische Orthesen Vorrichtung zum Trainieren der unteren Gliedmaße einer Person, insbesondere Personen mit Einschränkungen im Bereich des ZNS. Die Orthesen Vorrichtung bildet ein Interface mit zumindest einer unteren Gliedmaße aus und ist mit einer Stimulationsvorrichtung gekoppelt, die zumindest ein Elektrodenpaar aufweist, um auf den relevanten Muskel oder die relevante Muskelgruppe des Patienten einzuwirken. Die Orthese weist ein Gelenk auf, das mit einem Winkelsensor und zumindest einem Kraftsensor gekoppelt ist. Die Sensoren sind mit einer Steuerungsvorrichtung zur Steuerung der Stimulation gekoppelt. Das Gelenk ist zudem mit einem Motor zum Antreiben des Gelenkes gekoppelt, der durch ein Steuerungssystem in Echtzeit gesteuert wird.

Die US 2003/093021 A1 betrifft eine Vorrichtung zum Bewegen von Gelenken der unteren Extremität mit einer Orthese mit einer Vielzahl von gelenkig verbundenen Segmenten. Antriebsmittel sind ausgebildet, um eine Relativbewegung zwischen den Segmenten bereitzustellen. Zumindest ein Sensor ist ausgebildet, um die Winkelpositionen zumindest eines der Gelenksegmente zu erfassen. Eine Steuerungseinrichtung überwacht die Antriebsmittel und empfängt Rückkopplungsinformationen des zumindest einen Sensors, um es der Orthese zu ermöglichen, ein Gangmuster auszuführen. Die Vorrichtung weist weiterhin eine Einrichtung zur funktionalen Elektrostimulation auf.

Aufgabe der vorliegenden Erfindung ist es, ein unauffälliges orthopädietechnisches System bereitzustellen, mit dem das Bewegungsmuster eines Patienten, insbesondere das Gangbild eines Patienten, verbessert werden kann.

Erfindungsgemäß wird diese Aufgabe durch ein orthopädietechnisches System mit den Merkmalen des Anspruchs 1 gelöst. Vorteilhafte Ausgestaltungen und Weiterbildungen der Erfindung sind in der Beschreibung, den Unteransprüchen und den Figuren offenbart.

Ausgestaltungen, Ausführungen, Weiterbildungen und Beispiele, die nachstehend beschrieben werden aber nicht unter den Wortlaut der Ansprüche fallen, sind nicht Teil der Erfindung. Die nachstehend beschriebenen Verfahren sind auch nicht Teil der Erfindung.

Das orthopädietechnische System mit zumindest einer Stimulationselektrode zur Aktivierung zumindest eines Muskels, einer orthetischen oder prothetischen Einrichtung, die an einem Körper eines Patienten anlegbar und daran festlegbar ist, mit zumindest einem Gelenk, über das eine proximale und eine distale Komponente der orthetischen oder prothetischen Einrichtung schwenkbar miteinander verbunden sind, zumindest einer verstellbaren Widerstandseinrichtung, die zwischen der distalen Komponente und der proximalen Komponente angeordnet ist und über die ein Bewegungswiderstand gegen eine Verschwenkung der proximalen Komponente zu der distalen Komponente einstellbar ist, sieht Sensoren zur Erfassung von Kräften, Positionen, Beschleunigungen und/oder Momenten sowie eine Steuerungseinrichtung vor, die mit den Sensoren und der Widerstandseinrichtung gekoppelt ist, wobei die Steuerungseinrichtung Sensorwerte von den Sensoren verarbeitet und in Abhängigkeit von den Sensorwerten die Widerstandseinrichtung verstellt. Anders als bei motorisch angetriebenen Orthesen oder Prothesen ist es in dem erfindungsgemäßen orthopädietechnischen System vorgesehen, dass eine Stimulationselektrode einen Muskel stimuliert, beispielsweise über einen elektrischen Impuls, so dass aufgrund der daraufhin erfolgenden Muskelkontraktion eine verstärkte Dynamik der Bewegung oder überhaupt eine Bewegung der Gliedmaße oder der Restgliedmaße eingeleitet wird. Der aktivierte Muskel führt zu einer Erhöhung der Bewegungsenergie innerhalb des orthopädietechnischen Systems, was zu einer entsprechenden Bewegung der distalen und/oder proximalen Komponente des orthopädietechnischen Systems führt. Über die verstellbare Widerstandseinrichtung kann dann in Abhängigkeit von Sensorwerten, die einer Steuerungseinrichtung übermittelt werden, die wiederum mit der Widerstandseinrichtung gekoppelt ist, die Bewegung gesteuert oder geregelt werden, so dass das gewünschte Bewegungsmuster erreicht werden kann. Über das System ist es möglich, ohne einen aufwendigen motorischen Antrieb und ohne einen vergleichsweise schweren Energiespeicher Energie in das System einzubringen, indem die Muskulatur elektrisch aktiviert wird. Die Bewegung der Orthese oder Prothese wird über den verstellbaren Widerstand der Widerstandseinrichtung gesteuert, um eine Annäherung oder Anpassung an das gewünschte oder vorgegebene Bewegungsmuster zu erreichen. Der orthetischen oder prothetischen Einrichtung ist ein Energiespeicher zugeordnet, über den kinetische Energie der orthetischen oder prothetischen Einrichtung speicherbar und dieser kontrolliert wieder zuführbar ist. Über den Energiespeicher ist es möglich, dass kinetische Energie aus dem orthopädietechnischen System umgewandelt wird, beispielsweise wenn überschüssige Bewegungsenergie abgebaut werden muss, wobei durch die Speicherung der kinetischen Energie diese nicht dissipiert wird, insbesondere nicht vollständig in Wärme umgewandelt wird. Vielmehr ist es möglich, die kinetische Energie nach der Speicherung in einer anderen Energieform, beispielsweise als potentielle Energie durch Verformung einer Feder, durch Aufladen eines pneumatischen oder hydraulischen Druckspeichers oder durch Umwandlung in elektrische Energie, nach einer entsprechenden Umwandlung wieder dem orthopädietechnischen System zuzuführen. Damit ist es möglich dass die orthetische oder prothetische Einrichtung in ihrer Bewegung unterstützt werden kann, beispielsweise zur Unterstützung einer Flexion oder Extension der Komponenten zueinander, beispielsweise bei der Unterstützung des Aufstehens oder bei einem Anheben eines Gegenstandes. Alternativ erfolgt eine Umwandlung insbesondere dann, wenn ein erhöhter Widerstand gegen eine Bewegung aufzubringen ist, beispielsweise bei einem Abbremsen, sich Hinsetzen oder bei einem Gehen in der Standphasenflexion.

Die Stimulationselektrode kann als Oberflächenelektrode oder als Implantat ausgebildet sein. Die Wahl der Elektrodenform hängt von dem zu aktivierenden Muskel, von dem Verlauf eines zu stimulierenden Nerves oder der Art und Weise der Muskelaktivierung ab. Wird der Muskel über die Erregung eines noch vorhandenen Nerves aktiviert, der vergleichsweise nah an der Hautoberfläche verläuft, kann die Ausgestaltung der Stimulationselektrode als Oberflächenelektrode vorteilhaft sein, da eine solche Elektrode leicht anzubringen ist und keinen Implantationsaufwand zur Folge hat. Oberflächenstimulationselektroden können vergleichsweise einfach an der Hautoberfläche fixiert werden, haben jedoch den Nachteil, dass die Lokalisierung und damit auch die Aktivierung des jeweiligen Nerves unpräzise sein kann. Die Ausgestaltung der Elektrode als Implantat hat den Vorteil einer dauerhaften und präzisen Zuordnung der Elektrode zu dem jeweiligen Nerv oder Muskel, wenn dieser direkt aktiviert wird. Die implantierte Elektrode kann als sogenannte Cuff-Elektrode ausgebildet sein und sich manschettenartig um den anzuregenden Nerv legen, der zu dem zu stimulierenden Muskel führt, wenn der Muskel ober den ihn versorgenden Nerv aktiviert wird.

Die implantierte Stimulationselektrode kann als perkutane Stimulationselektrode ausgebildet sein, insbesondere für eine testweise Anwendung, bei der die Elektrode in unmittelbarer Nähe zu den anzuregenden Nervenästen implantiert wird. Die Elektrode kann neben der Ausgestaltung als Cuff-Elektrode auch als Elektrodenplatten mit mehreren Kontakten oder in einer anderen Art und Weise ausgestaltet sein. Die Anordnung erfolgt in unmittelbarer Nähe zu den Nervenästen, die jene Muskeln innervieren, die für die jeweilige Bewegung zuständig sind, beispielsweise für eine Dorsalflexion oder -extension eines Fußes, eine Unterschenkelflexion oder -extension oder eine Hüftflexion oder -extension. Ebenfalls ist es möglich, eine orthetische oder prothetische Einrichtung an einer oberen Extremität anzuordnen und eine entsprechende Stimulationselektrode in unmittelbarer Nähe zu denjenigen Nervenästen anzuordnen, die die zu aktivierenden Muskeln innervieren. Eine Elektrodenleitung führt von der Stimulationsstelle an den Nervenästen durch die Haut zu einem externen Impulsgeber, einem sogenannten Stimulator. Daneben können implantierte Stimulationselektroden auch als Hybridimplantat ausgebildet sein, bei der von der Elektrode ein Kabel zu einem ebenfalls implantierten Impulsgeber oder einem Empfänger führt, der unter der Hautoberfläche platziert ist. Dabei erfolgt kein Hautdurchtritt. Eine Energieversorgung für die Elektrode und eine Steuerung der Elektrodenimpulse erfolgt über eine direkt an der Hautoberfläche über dem Impulsgeber platzierten Sende- und Empfangseinheit, die für die Übertragung von Energie in Richtung auf den Impulsgeber und von Informationen auf den Impulsgeber und von dem Impulsgeber zu der Sende- und Empfangseinheit ausgebildet ist.

Eine weitere Variante der Ausgestaltung der Elektrode als Implantat sieht vor, die Elektrode, einen Impulsgeber, die Energieversorgung und die Steuereinrichtung gemeinsam zu implantieren. Ein solches implantierbares Modul kann selbstständig arbeiten und bedarf keiner weiteren Komponenten außerhalb des Moduls, nachdem die Steuerung einmal eingerichtet ist. Die Energieversorgung für die Steuereinrichtung und die Erzeugung eines Impulses findet entweder über eine Batterie oder einen Akku statt. Eine Verbindung von dem Implantat nach außen ist drahtlos ausgebildet, um Einstellungen vorzunehmen oder Daten auslesen zu können.

Eine Variante der Erfindung sieht vor, dass die Stimulationselektrode mit der Steuerungseinrichtung gekoppelt ist, entweder per Draht, drahtlos oder durch Integration der Steuerungseinrichtung in der Stimulationselektrode. Über die Steuerungseinrichtung werden der Zeitpunkt, die Art des Impulses, die Dauer des Impulses und die Impulsstärke festgelegt, mit denen der Nerv oder der Muskel direkt aktiviert oder stimuliert wird.

Der Energiespeicher kann als Federspeicher, Druckspeicher oder Speicher für elektrische Energie ausgebildet sein, wobei der orthetischen oder prothetischen Einrichtung ein Antrieb zugeordnet sein kann, um die gespeicherte Energie wieder in Bewegungsenergie umzuwandeln und eine Flexion oder Extension der distalen Komponente relativ zu der proximalen Komponente zu ermöglichen.

Die orthetische oder prothetische Einrichtung, die eine orthopädietechnische Einrichtung oder ein orthopädietechnisches System darstellt, kann als Orthese oder Prothese der unteren Extremität ausgebildet sein. Die Widerstandseinrichtung ist insbesondere als mechanische Bremse, als Fluiddämpfer, als magnetorheologischer Dämpfer oder als Generator ausgebildet, wobei die Widerstandseinrichtung durch eine Verstelleinrichtung, beispielsweise ein Aktuator, der mit der Steuerungseinrichtung gekoppelt ist, in Abhängigkeit von den Sensorwerten verstellt werden kann, um einen angepassten Widerstand bereitzustellen. Der Widerstand kann vergrößert oder verringert werden, wobei die Verstellung in Abhängigkeit von den erfassten Sensorgrößen wie Kräften, Positionen der distalen und/oder proximalen Komponenten in dem Raum oder zueinander, von Beschleunigungen, die die distalen und/oder proximalen Komponenten ausführen oder relativ zueinander ausführen und/oder Momenten, die auf die distalen und/oder proximalen Komponenten einwirken, erfolgen kann.

Das beschriebene Verfahren zur Steuerung eines orthopädietechnischen Systems, wie es oben beschrieben worden ist, sieht vor, dass über die zumindest eine Stimulationselektrode zumindest ein Muskel des Patienten aktiviert wird. Über Sensoren, die an der orthetischen oder prothetischen Einrichtung oder an dem Patienten angeordnet sind, können Kräfte, Positionen, Beschleunigungen und/oder Momente erfasst werden. Die Kräfte, Beschleunigungen und/oder Momente können auf die Komponenten der orthetischen oder prothetischen Einrichtung einwirken oder auch auf die Gliedmaße oder Gliedmaßen, an der oder denen das orthetische oder prothetische System befestigt ist. Die Positionen der Komponenten der orthetischen oder prothetischen Einrichtung sowie der jeweiligen Gliedmaßen können zu einer festen Bezugsgröße, beispielsweise zur Schwerkraftrichtung oder zueinander ermittelt werden. Die von den Sensoren erfassten Sensorwerte werden der Steuerungseinrichtung übermittelt, die in Abhängigkeit von den Sensorwerten eine Veränderung des Widerstandes in der Widerstandseinrichtung bewirkt. Dazu kann von der Steuerungseinrichtung ein entsprechendes Signal an eine Verstelleinrichtung übermittelt werden, die mit der Widerstandseinrichtung gekoppelt ist, um den Widerstand der Widerstandseinrichtung zu verringern oder zu vergrößern. Dazu können beispielsweise Ventile geöffnet oder geschlossen werden, um einen Fluidstrom mit einem verringerten oder vergrößerten Strömungswiderstand zu beaufschlagen. Ebenfalls kann über magnetische Erregung ein magnetorheologischer Dämpfer in dem von ihm aufgebrachten Widerstand verändert werden. Wird eine mechanische Bremse als Widerstandseinrichtung eingesetzt, kann eine Vergrößerung der Bremskraft, beispielsweise ein Anpressen von Bremsbelegen oder eine Verringerung eines Anpressdruckes einer Schlingfederbremse zu einer Anpassung der Widerstandseinrichtung führen. Bei einer Ausgestaltung der Widerstandseinrichtung als Generator kann durch Erregung von Spulen ein erhöhter Widerstand gegen eine Rotation bereitgestellt werden.

Eine Weiterbildung der Erfindung sieht vor, dass über zumindest zwei Stimulationselektroden zueinander antagonistisch wirkende Muskeln zeitversetzt aktiviert werden, um eine Flexion und Extension der mit den Muskeln gekoppelten Gliedmaße zu bewirken. Der Zeitversatz kann in der Steuerungseinrichtung fest einprogrammiert sein. Alternativ wird die wechselseitige Stimulation über Sensoren gesteuert, die mit der Steuerungseinrichtung gekoppelt sind und in Abhängigkeit von den erfassten Bewegungsparametern oder Belastungsparametern eine entsprechende Stimulierung durchführen.

Eine Weiterbildung der Erfindung sieht vor, dass eine der orthetischen oder prothetischen Einrichtung zugeordneter Antrieb eine Verschwenkbewegung der proximalen Komponente relativ zu der distalen Komponente unterstütz, beispielsweise um ein Anheben eines Gegenstandes zu erleichtern oder einem Patienten bei einem Aufstehen zu helfen.

Die Bewegungsenergie aus der aktivierten Muskulatur kann bei einem Abbremsen der Bewegung der orthetischen oder prothetischen Einrichtung in einem Energiespeicher gespeichert und zum Antreiben der orthetischen oder prothetischen Einrichtung zeitversetzt wieder zugeführt werden. Dadurch ist es möglich, dass die Muskulatur nicht bei jeder Bewegung der orthetischen oder prothetischen Einrichtung aktiviert werden muss oder die Aktivierung auf einem niedrigeren Erregungsniveau stattfinden kann. Darüber hinaus ist es möglich, dass bei einer Umwandlung der kinetischen Energie in elektrischer Energie diese zur Energieversorgung der Stimulationselektroden und der Steuerungseinrichtung verwendet wird.

Eine Muskelaktivierung kann in Abhängigkeit von einer auf einer Gliedmaße oder einer Komponente der orthetischen oder prothetischen Einrichtung ausgeübten Kraft oder von einem auf eine Gliedmaße oder eine Komponente der orthetischen oder prothetischen Einrichtung ausgeübten Moment erfolgen. Je nach Größe der Belastung ist es somit möglich, eine angepasste Muskelaktivierung zu bewirken. Die auf die orthetische oder prothetische Einrichtung ausgeübte Kraft bzw. das ausgeübte Moment oder die auf die Gliedmaßen ausgeübten Kräfte oder Momente stellen Bewegungsindikatoren dar, die Rückschlüsse darauf geben, ob und inwieweit eine Muskelaktivierung erfolgen soll.

Eine entsprechende Muskelaktivierung kann in Abhängigkeit von einer von einer Gliedmaße oder einer Komponente der orthopädischen oder auch prothetischen Einrichtung ausgeführten Beschleunigung und/oder in Abhängigkeit von der Position einer Gliedmaße oder einer Komponente der orthetischen oder prothetischen Einrichtung in dem Raum oder zueinander erfolgen. Ebenfalls ist es möglich, eine Kombination davon der Muskelaktivierung zugrunde zu legen, beispielsweise in Abhängigkeit von einem auf eine Komponente ausgeübten Moment in Abhängigkeit von der gegenwärtigen Position der Komponente in dem Raum oder relativ zu einer anderen Komponente.

Insbesondere ist vorgesehen, dass eine Aktivierung der Hüftmuskulatur und/oder der Oberschenkelmuskulatur erfolgt, um eine Verbesserung des Gangverhaltens und der Mobilität eines Patienten sicherzustellen.

Nachfolgend werden Ausführungsbeispiele der Erfindung anhand der beigefügten Figuren näher erläutert. Es zeigen:
- Figur 1-: eine Variante der Erfindung als Orthese; sowie
- Figur 2 -: eine Variante der Erfindung als Prothese.

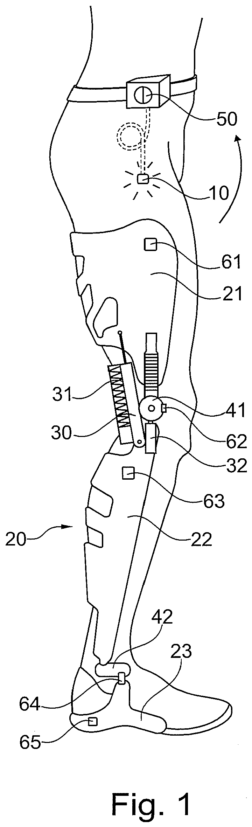

Figur 1 zeigt ein orthopädietechnisches System mit einer Stimulationselektrode 10, die in dem dargestellten Ausführungsbeispiel als implantierbare Elektrode ausgebildet ist und implantiert ist. Die Stimulationselektrode 10 dient zur Aktivierung eines Hüftbeugermuskels oder einer Gruppe von Muskeln, die eine Hüftbeugung bewirken. Die Stimulationselektrode 10 ist in dem dargestellten Ausführungsbeispiel als perkutane Stimulationselektrode ausgebildet, die in Gestalt einer Manschette oder in Gestalt von Elektrodenplatten mit mehreren Kontakten in unmittelbarer Nähe zu Nervenästen angeordnet ist, die den zumindest einen Muskel oder die Muskelgruppe innervieren, die für die Hüftbeugung verantwortlich sind. Die Stimulationselektrode 10 kann neben der Aktivierung des Muskels über den ihn versorgenden Nerv oder die ihn versorgenden Nerven auch direkt den Muskel aktivieren, wozu eine direkte Kopplung der Elektrode mit dem Muskel notwendig und vorgesehen ist. Bei der perkutanen Stimulationselektrode 10 führt eine Elektrodenleitung von der Stimulationsstelle durch die Haut zu einem externen Impulsgeber oder der Steuereinrichtung 50, die nicht implantiert und außerhalb des Körpers an dem Patienten befestigt ist. Die Kopplung der Elektrodenleitung mit der Steuerungseinrichtung 50 kann über einen lösbaren Stecker oder eine magnetische Kontaktfläche erfolgen, so dass eine Trennung der Stimulationselektrode 10 von der Steuerungseinrichtung 50 problemlos möglich ist.

Die Stimulationselektrode 10 kann auch als Hybridimplantat ausgebildet sein, bei dem von der Elektrode 10 ein Kabel zu einem implantierten Impulsgeber oder einer Empfängerfläche führt, der oder die in der Hautoberfläche angeordnet ist. Die Energieversorgung ebenso wie die Steuerung der Elektrode zur Aktivierung des Muskels entweder direkt oder über den den Muskel versorgenden Nerv erfolgt über die externe Steuerungseinrichtung 50, die eine direkt an der Hautoberfläche über dem Impulsgeber oder der Kontaktfläche angeordneten Sende- und Empfangseinheit aufweist, die per Kabel oder Funk mit der Steuerungseinrichtung 50 gekoppelt ist. Über die Sende- und Empfangseinheit erfolgt die Übertragung von Energie und Informationen.

Alternativ zu der Anordnung der Steuerungseinrichtung 50 extern an dem Patienten kann in einer Variante der Erfindung die Elektrode zusammen mit dem Impulsgeber, der Energieversorgung und der Steuerungseinrichtung als gemeinsames Vollimplantat ausgebildet sein, das autonom arbeitet. Eine drahtlose Verbindung von dem Implantat nach außen ist vorgesehen, um Einstellungen vornehmen oder Daten auslesen zu können. Auch hier kann die Aktivierung des Muskels entweder direkt oder über die Stimulierung des den Muskel versorgenden Nerves über die Elektrode erfolgen. Das Vollimplnatat kann gleichzeitig mit einer Sensoreinrichtung versehen sein, um über die Aktivierung des Muskels und/oder die Position des Implantats in dem Raum oder über einwirkende Kräfte und/oder Momente Sensordaten zu erhalten.

Als zweite Komponente des orthopädietechnischen Systems ist die orthetische Einrichtung 20 in Gestalt einer KAFO (knee ankle foot orthosis) ausgebildet, also einer Orthese, die sich von dem Oberschenkel bis zum Fuß erstreckt und das Kniegelenk und das Knöchelgelenk überbrückt. Die orthetische Einrichtung 20 weist in dem dargestellten Ausführungsbeispiel eine proximale Komponente 21 in Gestalt einer Oberschenkelschiene oder einer Oberschenkelschale auf, die über ein orthetisches Kniegelenk 41 mit einer distalen Komponente 22 in Gestalt einer Unterschenkelschiene oder Unterschenkelschale schwenkbar gekoppelt ist. Weiter distal in an der Unterschenkelkomponente 22 eine Fußkomponente 23 in Gestalt einer Fußschale oder einer Fußschiene angeordnet, die über ein orthetisches Knöchelgelenk 42 mit der Unterschenkelschiene 22 verbunden ist. Die Unterschenkelschiene 22 kann auch die proximale Komponente ausbilden, wenn keine Oberschenkelkomponente 21 daran befestigt ist. Es handelt sich dann nur noch um eine AFO (ankle foot orthosis), wobei zwischen dem Fußteil und der Fußschale 23 als distale Komponente und der dann proximalen Komponente 22 als Unterschenkelschiene oder Unterschenkelschale eine nicht dargestellte Widerstandseinrichtung angeordnet sein kann.

In dem dargestellten Ausführungsbeispiel ist eine Widerstandseinrichtung 30 zwischen der proximalen Komponente 21 oder Oberschenkelschiene oder Oberschenkelschale und der distalen Komponente 22 oder Unterschenkelschiene oder Unterschenkelschale angeordnet und jeweils an einer Komponente 21, 22 befestigt. Die Widerstandseinrichtung 30 kann einen Widerstand gegen eine Flexion oder Extension der proximalen Komponente 21 relativ zu der distalen Komponente 22 bereitstellen. Die Widerstandseinrichtung 30 kann auch als Sperreinrichtung ausgebildet sein und jegliche Verschwenkung der distalen Komponente 22 zu der proximalen Komponente 21 verhindern. Hierzu ist eine nicht dargestellte Verstelleinrichtung der Widerstandseinrichtung 30 zugeordnet, über die der Widerstand angepasst oder maximal erhöht werden kann, so dass in dem Rahmen der üblicherweise bei der Benutzung einer Orthese der unteren Extremität auftretenden Belastung keine Relativbewegung um die Gelenkachse des orthetischen Kniegelenkes 41 möglich ist. Die Widerstandseinrichtung kann als Fluiddämpfer, insbesondere als Hydraulikdämpfer ausgebildet sein, ebenso kann eine mechanische Bremse, eine magnetorheologische Widerstandseinrichtung oder ein Generator als Widerstandseinrichtung 30 zwischen den Komponenten 21, 22 angeordnet sein, um einen angepassten Widerstand gegen eine Verschwenkbewegung bereitzustellen.

Der Widerstandseinrichtung 30 zugeordnet ist ein Energiespeicher 31, der schematisch dargestellt ist und als Federspeicher oder Druckspeicher ausgebildet sein kann. Grundsätzlich ist es auch möglich, die Energiespeichereinrichtung 31 als Speicher für elektrische Energie auszugestalten, um Bewegungsenergie in elektrische Energie umwandeln zu können. Bei der Ausgestaltung der Energiespeichereinrichtung 31 als mechanischer Energiespeicher wird die Bewegungsenergie, die bei einer Flexion und/oder Extension der Komponenten 21, 22 relativ zueinander aufgebracht wird, in potentielle Energie umgewandelt. Über den Energiespeicher 31 kann zeitlich abgestimmt über entweder die Steuerungseinrichtung 50 oder über eine gesonderte, mit der Steuerungseinrichtung 50 gekoppelte Orthesensteuerung Energie freigegeben werden, um eine Flexion oder Extension der Komponenten 21, 22 zueinander zu unterstützen. Dies erfolgt über einen schematisch dargestellten motorischen Antrieb 32, der von dem Energiespeicher 31 mit Energie versorgt wird und eine Verschwenkbewegung in die eine oder andere Richtung bewirkt oder zumindest unterstützt.

Eine korrespondierende Ausgestaltung mit einer Widerstandseinrichtung 30, Energiespeicher 31 und Antrieb oder Motor 32 kann auch zwischen der Unterschenkelkomponente 22 und der Fußkomponente 23 angeordnet sein, um so einen gewünschten oder benötigten Widerstand gegen eine Dorsalflexion oder Dorsalextension des Fußes bereitzustellen oder aber eine entsprechende Antriebsleistung und eine Verschwenkbewegung über den Energiespeicher 31 und den Antrieb 32 zu bewirken. Statt einer Verschwenkung des Unterschenkelteils 22 relativ zu dem Oberschenkelteil 21 erfolgt dann eine Verschwenkung des Fußteils 23 relativ zu dem Unterschenkelteil 22. Grundsätzlich ist es auch möglich, zwei Widerstandseinrichtungen 30 mit einem oder zwei Energiespeichern 31 und einem oder zwei Antrieben 32 vorzusehen, um beide Gelenke 41, 42 zu überbrücken, mehrere Komponenten anzutreiben und abzubremsen und eine Verbesserung des Gangbildes oder eine Anpassung des Gangbildes an ein gewünschtes Gangbild zu ermöglichen.

In der orthetischen Einrichtung 20 sind in dem dargestellten Ausführungsbeispiel fünf Sensoren 61, 62, 63, 64, 65 angeordnet, die mit der orthetischen Einrichtung 20 und der Steuerungseinrichtung 50 gekoppelt sind.

An der Oberschenkelkomponente 21 ist ein erster Sensor 61 angeordnet, der drahtlos mit der Steuerungseinrichtung 50 gekoppelt ist und die Position oder Winkelstellung der Oberschenkelkomponente 21 und damit auch des Oberschenkels bei angelegter Ortheseneinrichtung 20 detektiert. Der Sensor 61 kann als Gyroskop oder anderer Lagesensor ausgebildet sein, um die Orientierung der Oberschenkelkomponente 21 und des Oberschenkels relativ zu der Vertikalen zu detektieren.

Im Bereich des orthetischen Kniegelenkes 41 ist ein weiterer Sensor 62 angeordnet, der als Winkelsensor ausgebildet ist, um die Winkelstellung der Oberschenkelkomponente 21 relativ zu der Unterschenkelkomponente 22 zu ermitteln. Der Sensor kann als inkrementaler Sensor oder Absolutsensor ausgebildet sein. Er kann als optischer Sensor ausgebildet sein oder auf einer magnetischen Wirkungsweise basieren, beispielsweise als Halleffektsensor.

An der Unterschenkelkomponente 22, also derjenigen Komponente, die distal zu der Oberschenkelkomponente 21 über das Orthesenkniegelenk 41 schwenkbar befestigt ist, ist in dem Bereich des orthetischen Kniegelenkes 41 ein weiterer Sensor 63 angeordnet, der entweder als Lagesensor analog zum ersten Lagesensor 61 oder als Momentensensor oder eine Kombination davon ausgebildet ist, um entweder die absolute Position der Unterschenkelkomponente 22 und/oder die auf die Unterschenkelkomponente 22 wirkenden Belastungen erfassen zu können. Grundsätzlich ist es möglich, dass auch in dem Winkelsensor 62 zur Ermittlung des Kniewinkels eine weitere Sensorkomponente angeordnet ist, beispielsweise um die in dem Kniegelenk 41 wirkenden Momente oder Kräfte erfassen zu können.

Distal zu der Unterschenkelkomponente 22 ist ein Fußteil 23 über das Knöchelgelenk 42 schwenkbar befestigt. Das Knöchelgelenk 42 befindet sich auf der Höhe des natürlichen Knöchelgelenkes, entsprechend befindet sich das orthetische Kniegelenk 41 in dem Bereich des natürlichen Kniegelenkes oder der natürlichen Kniegelenkachse. Das Fußteil 23 untergreift den Fuß und hat eine Sohlenkomponente, so dass der Fuß in Dorsalflexions- und Plantarflexionsrichtung geführt und unterstützt ist. An dem Knöchelgelenk 42 ist ein weiterer Sensor 64 angeordnet, der die Winkelstellung des Fußteils 23 relativ zu dem Unterschenkelteil 22 und/oder die um das Knöchelgelenk 42 wirkenden Momente erfasst, um so Rückschlüsse auf die gegenwärtige Belastung und die Bewegungssituation oder Gangsituation erhalten zu können. An der Unterseite des Fußteils 23 ist ein Kraftsensor 65 oder Drucksensor angeordnet, um den Fersenstoß oder Fersenkontakt zu detektieren. Ein solcher Fersenschalter 65 lässt Rückschlüsse auf die Gangsituation, den Verlauf der Gangsituation sowie die gegenwärtige Bewegungssituation zu. Wird nur eine geringe Belastung über einen längeren Zeitraum unverändert detektiert, kann davon ausgegangen werden, dass sich der Träger der orthetischen Einrichtung 20 in einer sitzenden Stellung befindet. Bei einem sich verändernden Belastungswert in dem Fersenbereich mit einer vollständigen Entlastung kann auf ein Gehen geschlossen werden. Über die Höhe der Belastung, den Ablauf der Belastung und das Intervall von Belastung und Entlastung können Rückschlüsse auf die Art der Bewegung und mit welcher Ganggeschwindigkeit gegangen wird gezogen werden.

Alle Werte der Sensoren 61 - 64 werden der Steuerungseinrichtung 50 und/oder einer separaten, nicht dargestellten zweiten Steuerungseinrichtung übermittelt, die auf Grundlage der Sensordaten eine Veränderung des Flexionswiderstandes und/oder Extensionswiderstandes in dem Kniegelenk 41 und/oder Knöchelgelenk 42 vornimmt oder vornehmen. Ebenso kann das Speichern von Energie in dem Energiespeicher 31 sowie die Abgabe der Energie aus dem Energiespeicher an den Antrieb 32 über die Steuerungseinrichtung veranlasst werden, so dass eine Bewegungsunterstützung sowie eine Energiespeicherung zur Verringerung der aufzubringenden Bewegungsenergie durch einen aktivierten Hüftbeugermuskel zu bewirken.

Die Steuereinrichtung 50 enthält auch einen Stimulator zur Erzeugung eines Stimulationsimpulses oder der Stimulationsimpulse.

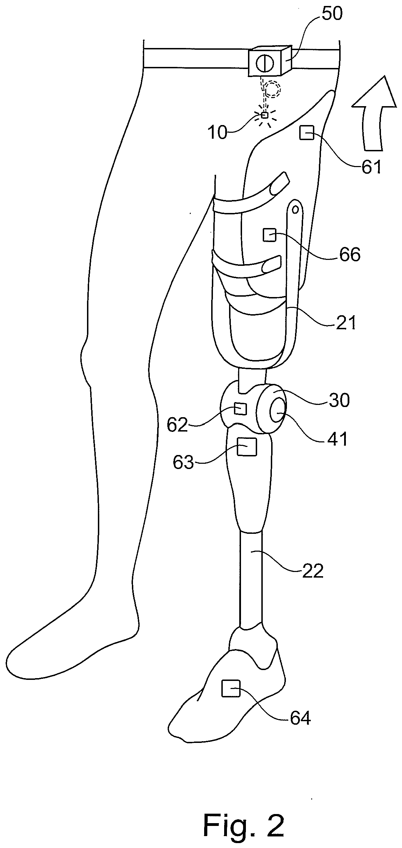

Eine Variante der Erfindung ist in der Figur 2 dargestellt, bei der statt einer Orthese eine Prothese vorhanden ist. Auch hier befindet sich eine externe Steuerungseinrichtung 50 außerhalb des Körpers und ist an einem Gürtel an dem Patienten befestigt. Ein erster Positionssensor 61 ist an einer Oberschenkelkomponente in Gestalt eines Oberschenkelschaftes 21, der über eine Spange mit einem Prothesenkniegelenk 41 verbunden ist, gekoppelt. Der Lagesensor 61 ist in dem dargestellten Ausführungsbeispiel per Kabel mit der Steuerungseinrichtung 50 gekoppelt. Ein Lagesensor 66 an einem Oberschenkelschaft 21 kann per Funk mit der Steuerungseinrichtung 50 gekoppelt sein, um eine präzise Information über die gegenwärtige Lage des Oberschenkelschaftes 21 sowie des Oberschenkelstumpfes bereitstellen zu können.

An dem distalen Ende der Oberschenkelkomponente 21 ist ein Prothesenkniegelenk 41 angeordnet, das die proximale Oberschenkelkomponente 21 mit der distalen Unterschenkelkomponente 22 schwenkbar verbindet. Innerhalb des Prothesenkniegelenkes 41 ist eine verstellbare Widerstandseinrichtung 30 angeordnet, die beispielsweise als mechanische Bremse, Hydraulikdämpfer, Pneumatikdämpfer, magnetorheologischer Dämpfer oder Generator ausgebildet sein kann. Ein nicht dargestellter Energiespeicher in Gestalt einer Feder, eines Druckbehälters oder eines Akkus kann ebenfalls in dem Prothesenkniegelenk 41 integriert sein. Auch ein nicht dargestellter Antrieb, beispielsweise ein Federantrieb oder ein Elektromotor, kann in dem Prothesenkniegelenk 41 integriert sein, um neben einer Energiespeicherung bei einem Abbremsen beispielsweise der Unterschenkelkomponente 22 relativ zu der Oberschenkelkomponente 21 Energie nicht in Wärme umzuwandeln, sondern sinnvoll zu speichern, damit diese wieder über den Antrieb zur Extension oder Flexion der Unterschenkelkomponente 22 relativ zu der Oberschenkelkomponente 21 zur Verfügung gestellt werden kann.

An dem Prothesenkniegelenk 41 ist ein Sensor 62 zur Erfassung des Kniewinkels und/oder des auf das Kniegelenk 41 wirkenden Momentes angeordnet. Distal zu dem Prothesenkniegelenk 41 ist ein weiterer Sensor 63 an der Unterschenkelkomponente 22 angeordnet, um die Lage der Unterschenkelkomponente 22 relativ zu der Oberschenkelkomponente 21 oder absolut zu der Vertikalen erfassen zu können. An dem Prothesenfuß ist ein weiterer Sensor 64 zur Erfassung der auf den Prothesenfuß wirkenden Axialkraft oder eines Momentes angeordnet. Alle Sensoren 61, 62, 63, 64, 66 sind mit der Steuerungseinrichtung 50 gekoppelt, die wiederum mit der Stimulationselektrode 10 zur Stimulation zumindest eines Muskels, in dem dargestellten Ausführungsbeispiel des Hüftebeugermuskels, gekoppelt ist. Darüber hinaus kann die Steuerungseinrichtung 50 auch die Widerstandseinrichtung 30 innerhalb des Prothesenkniegelenkes 41 steuern, um einen erhöhten oder verringerten Flexions- oder Extensionswiderstand in Abhängigkeit von den ermittelten Sensorwerten bereitzustellen.

Sowohl die orthetische Einrichtung 20 als auch die prothetische Einrichtung 20 funktioniert nach dem Prinzip, dass über die Steuerungseinrichtung 50 zumindest eine Stimulationselektrode 10 angesteuert wird und mit einem Stimulationsimpuls versehen wird, um den jeweiligen Muskel zur Kontraktion anzuregen. In dem dargestellten Ausführungsbeispiel wird ein Hüftbeugermuskel oder eine Hüftbeugermuskelgruppe durch die Stimulationselektrode 10 entweder direkt oder über den Muskel oder die Muskelgruppe versorgenden Nerv aktiviert, so dass eine Hüftbeugung entsprechend dem Pfeil aktiviert. Über die Hüftbeugung wird in das orthetische oder prothetische System 20 ein Energieimpuls eingebracht, mit dem der Unterschenkel 22 bzw. die Unterschenkelkomponente 22 nach vorn in Gehrichtung bewegt wird. Dementsprechend wird auch das Fußteil 23 oder der Prothesenfuß nach vorne bewegt, so dass eine Extensionsbewegung in dem Orthesen- oder Prothesenkniegelenk 41 stattfindet. Da die Steuerung der Muskelimpulse unter Umständen nicht ausreichend präzise sein kann, wird erfindungsgemäß die Flexionsbewegung und Extensionsbewegung der distalen Komponente 22 oder der Unterschenkelkomponente relativ zu der proximalen Komponente 21 oder Oberschenkelkomponente über die Widerstandseinrichtung 30 korrigiert, wobei die Widerstandseinrichtung 30 in Abhängigkeit von einer Vielzahl von Sensorwerten oder auch nur eines Sensorwertes verstellt wird. Wird über die Muskelaktivierung zu viel Energie in die orthetische oder prothetische Einrichtung 20 eingebracht, wird über die Widerstandseinrichtung 30 ein erhöhter Widerstand bereitgestellt. Entsprechend wird bei einer verringerten oder zu geringen Energieeinbringung aufgrund einer schwächeren Muskelkontraktion der Widerstand entsprechend verringert oder der Endanschlag dergestalt verstellt, dass ein harmonisches oder ein an ein vorgegebenes Gangbild angepasstes Gangbild erreicht wird.

Die orthetische oder prothetische Einrichtung 20 kann synergistisch oder antagonistisch mit der stimulierten Muskulatur zusammenwirken. Neben der Stimulation der Hüftbeugermuskulatur kann auch eine Stimulation der Hüftstreckmuskulatur stattfinden. Ebenso ist es möglich, dass die Hüftbeugung durch den musculus sartorius, dem musculus rectus femoris und den musculus iliacus sowie den musculus psoas aktiviert wird. Der Hüftbeugebewegung entgegenwirkend kann dann die Hüftstreckung über den musculus gluteus maximus aktiviert werden. Durch eine zeitlich gesteuerte Aktivierung der jeweiligen Muskeln kann eine abgestimmte Hüftbeugung und Hüftstreckung erreicht werden. Ebenso kann auf Grundlage der Sensoren nach einer Aktivierung beispielsweise der Hüftbeugung zur Begrenzung der Hüftbeugebewegung eine Aktivierung der Hüftstreckmuskulatur eingesetzt werden.

Alternativ oder ergänzend kann bei einer Stimulation der Oberschenkelmuskulatur zur Knieextension der musculus quadriceps aktiviert und zur Kniebeugung der musculus biceps femoris, der musculus semitendinosus sowie der musculus semimembranosus aktiviert werden. Auch hier kann ergänzend zu der Widerstandseinrichtung 30 bei einer orthetischen Versorgung eine abgestimmte Aktivierung synergistischer und antagonistisch wirkender Muskeln zusammen mit der Widerstandseinrichtung 30 erfolgen. Die Bewegungsenergie aus der stimulierten Muskulatur wird in der orthetischen oder prothetischen Einrichtung über den Energiespeicher 31 zwischengespeichert und zur Unterstützung, also synergistisch zu der stimulierten Muskulatur, über den Antrieb 32 wieder dem System zugeführt.

Die Aktivierung oder Stimulation der Muskulatur erfolgt über ein Muskelinterface oder ein Nerveninterface. Neben einer Stimulation über ein implantiertes System, wie in den Ausführungsbeispielen beschrieben, kann diese auch über ein Oberflächenstimulationssystem mit Oberflächenelektroden erfolgen, sofern die Muskeln oder Nerven von außen zugänglich sind. Über die Widerstandseinrichtung 30 in Verbindung mit der Steuerungseinrichtung 50 und den Sensoren 61 - 66 kontrolliert das mechanische System der prothetischen und orthetischen Einrichtung 20 die Bewegungsausführung durch die Anpassung der Dämpfung über die Widerstandseinrichtung 30 und der Sperrung oder der Verstellung von Streckanschlägen und Beugeanschlägen zur Eingrenzung der maximal erreichbaren Winkelstellungen.

## Patentansprüche

1. Orthopädietechnisches System mit
a. zumindest einer Stimulationselektrode (10) zur Aktivierung zumindest eines Muskels,
b. einer orthetischen oder prothetischen Einrichtung (20), die an einem Körper eines Patienten anlegbar und daran festlegbar ist, mit zumindest einem Gelenk (41, 42), über das eine proximale und eine distale Komponente (21, 22; 22, 23) der orthetischen oder prothetischen Einrichtung (20) schwenkbar miteinander verbunden sind,
c. zumindest einer verstellbaren Widerstandseinrichtung (30), die zwischen der distalen und der proximalen Komponente (21,22) angeordnet ist und über die ein Bewegungswiderstand gegen eine Verschwenkung der proximalen Komponente (21) zu der distalen Komponenten (22) einstellbar ist,
d. Sensoren (61, 62, 63, 64, 65, 66) zur Erfassung von Kräften, Positionen, Beschleunigungen und/oder Momenten,
e. einer Steuerungseinrichtung (50), die mit den Sensoren (61, 62, 63, 64, 65, 66) und der Widerstandseinrichtung (30) gekoppelt ist, Sensorwerte von den Sensoren (61, 62, 63, 64, 65, 66) verarbeitet und in Abhängigkeit von den Sensorwerten die Widerstandseinrichtung (30) verstellt,
f. **dadurch gekennzeichnet, dass** der orthetischen oder prothetischen Einrichtung (20) ein Energiespeicher (31) zugeordnet ist, über den kinetische Energie der orthetischen oder prothetischen Einrichtung (20) speicherbar und kontrolliert wieder zuführbar ist.

2. Orthopädietechnisches System nach Anspruch 1, **dadurch gekennzeichnet, dass** die Stimulationselektrode (10) als Oberflächenelektrode oder als Implantat ausgebildet ist.

3. Orthopädietechnisches System nach Anspruch 1 oder 2, **dadurch gekennzeichnet, dass** die Stimulationselektrode (10) mit der Steuerungseinrichtung (50) gekoppelt ist.

4. Orthopädietechnisches System nach einem der voranstehenden Ansprüche, **dadurch gekennzeichnet, dass** der Energiespeicher (31) als Federspeicher, Druckspeicher oder Speicher für elektrische Energie ausgebildet ist.

5. Orthopädietechnisches System nach einem der voranstehenden Ansprüche, **dadurch gekennzeichnet, dass** der orthetischen oder prothetischen Einrichtung (20) ein Antrieb (32) zugeordnet ist.

6. Orthopädietechnisches System nach einem der voranstehenden Ansprüche, **dadurch gekennzeichnet, dass** die orthetische oder prothetische Einrichtung (20) als Orthese oder Prothese der unteren Extremität ausgebildet ist.

7. Orthopädietechnisches System nach einem der voranstehenden Ansprüche, **dadurch gekennzeichnet, dass** die Widerstandseinrichtung (30) als mechanische Bremse, als Fluiddämpfer, als magnetorheologischer Dämpfer oder als Generator ausgebildet ist.

8. Orthopädietechnisches System nach Anspruch 1, **dadurch gekennzeichnet, dass** das System derart ausgelegt ist, dass über zumindest zwei Stimulationselektroden (10) zueinander antagonistisch wirkende Muskeln zeitversetzt aktiviert werden.

9. Orthopädietechnisches System nach Anspruch 8, **dadurch gekennzeichnet, dass** ein der orthetischen oder prothetischen Einrichtung (20) zugeordneter Antrieb (32) eine Verschwenkbewegung der proximalen Komponente (21) relativ zu der distalen Komponente (22) unterstützt.

10. Orthopädietechnisches System nach einem der Ansprüche 1 bis 9, **dadurch gekennzeichnet, dass** das System derart ausgelegt ist, dass eine Muskelaktivierung in Abhängigkeit von einer auf eine Gliedmaße oder eine Komponente (21, 22, 23, 41, 42) der orthetischen oder prothetischen Einrichtung (20) ausgeübten Kraft oder von einem auf eine Gliedmaße oder eine Komponente (21, 22, 23, 41, 42) der orthetischen oder prothetischen Einrichtung (20) ausgeübten Moment erfolgt.

11. Orthopädietechnisches System nach einem der Ansprüche 1 bis 10, **dadurch gekennzeichnet, dass** das System derart ausgelegt ist, dass eine Muskelaktivierung in Abhängigkeit von einer von einer Gliedmaße oder einer Komponente (21, 22, 23, 41, 42) der orthetischen oder prothetischen Einrichtung (20) ausgeführten Beschleunigung und/oder in Abhängigkeit von der Position einer Gliedmaße oder einer Komponente (21, 22, 23, 41, 42) der orthetischen oder prothetischen Einrichtung (20) im Raum oder zueinander erfolgt.

12. Orthopädietechnisches System nach einem der Ansprüche 1 bis 11, **dadurch gekennzeichnet, dass** das System derart ausgelegt ist, dass eine Aktivierung der Hüftmuskulatur und/oder der Oberschenkelmuskulatur erfolgt.

## Claims

1. Orthopaedic system with
a) at least one stimulation electrode (10) for activating at least one muscle,
b) an orthotic or prosthetic device (20) which can be placed on a patient's body and secured there, with at least one joint (41, 42) by which a proximal and a distal component (21, 22; 22, 23) of the orthotic or prosthetic device (20) are connected to each other in swivelling fashion,
c) at least one adjustable resistance device (30) which is arranged between the distal component and the proximal component (21, 22) and with which a motion resistance against a swivelling of the proximal component (21) relative to the distal component (22) is adjustable,
d) sensors (61, 62, 63, 64, 65, 66) for detecting of forces, positions, accelerations and/or torques,
e) a control device (50) which is coupled to the sensors (61, 62, 63, 64, 65, 66) and the resistance device (30), processes sensor values of the sensors (61, 62, 63, 64, 65, 66) and adjusts the resistance device (30) in dependence on the sensor values
f) **characterized in that** the orthotic or prosthetic device (20) is associated with an energy accumulator (31), by which kinetic energy of the orthotic or prosthetic device (20) is storable and returnable to it in a controlled manner.

2. Orthopaedic system according to Claim 1, **characterized in that** the stimulation electrode (10) is designed as a surface electrode or as an implant.

3. Orthopaedic system according to Claim 1 or 2, **characterized in that** the stimulation electrode (10) is coupled to the control device (50).

4. Orthopaedic system according to one of the preceding claims, **characterized in that** the energy accumulator (31) is designed as a spring accumulator, pressure accumulator, or accumulator of electric energy.

5. Orthopaedic system according to one of the preceding claims, **characterized in that** the orthotic or prosthetic device (20) is associated with a drive unit (32).

6. Orthopaedic system according to one of the preceding claims, **characterized in that** the orthotic or prosthetic device (20) is designed as an orthotic or prosthesis of the lower limb.

7. Orthopaedic system according to one of the preceding claims, **characterized in that** the resistance device (30) is formed as a mechanical brake, a fluid damper, a magnetorheological damper, or a generator.

8. Orthopaedic system according to claim 1, **characterized in that** the system is configured that muscles which act antagonistically to each other are activated by means of at least two stimulation electrodes (10) delayed in time.

9. Orthopaedic system according to Claim 8, **characterized in that** a drive unit (32) which is associated with the orthotic or prosthetic device (20) assists a swivelling movement of the proximal component (21) relative to the distal component (22).

10. Orthopaedic system according to one of Claims 1 to 9, **characterized in that** the system is configured that a muscle activation occurs in dependence on a force exerted on a limb or a component (21, 22, 23, 41, 42) of the orthotic or prosthetic device (20) or a torque exerted on a limb or a component (21, 22, 23, 41, 42) of the orthotic or prosthetic device (20).

11. Orthopaedic system according to one of Claims 1 to 10, **characterized in that** the system is configured that a muscle activation occurs in dependence on an acceleration performed by a limb or a component (21, 22, 23, 41, 42) of the orthotic or prosthetic device (20) and/or in dependence on the position of a limb or a component (21, 22, 23, 41, 42) of the orthotic or prosthetic device (20) in space or relative to each other.

12. Orthopaedic system according to one of Claims 1 to 11, **characterized in that** the system is configured that an activation of the hip muscles and/or the thigh muscles occurs.

## Revendications

1. Système orthopédique comportant
a. au moins une électrode de stimulation (10) pour activer au moins un muscle,
b. un ensemble formant orthèse ou prothèse (20) pouvant être appliqué et immobilisé au corps d'un patient, présentant au moins une articulation (41, 42), par laquelle un composant proximal et un composant distal (21, 22 ; 22, 23) de l'ensemble formant orthèse ou prothèse (20) sont reliés entre eux de manière pivotante,
c. au moins un dispositif de résistance réglable (30) qui est disposé entre les composants distal et proximal (21, 22) et qui permet de régler une résistance au mouvement à l'encontre d'un pivotement du composant proximal (21) par rapport au composant distal (22),
d. des capteurs (61, 62, 63, 64, 65, 66) pour l'enregistrement des forces, positions, accélérations et/ou couples,
e. un dispositif de commande (50) qui est couplé aux capteurs (61, 62, 63, 64, 65, 66) et au dispositif de résistance (30), qui traite les valeurs des capteurs (61, 62, 63, 64, 65, 66) et qui ajuste le dispositif de résistance (30) en fonction des valeurs des capteurs,
f. **caractérisé en ce que**
un accumulateur d'énergie (31) est associé à l'ensemble formant orthèse ou prothèse (20), permettant d'accumuler de l'énergie cinétique de l'ensemble formant orthèse ou prothèse (20) et de la restituer de façon contrôlée.

2. Système orthopédique selon la revendication 1,
**caractérisé en ce que** l'électrode de stimulation (10) est réalisée comme une électrode de surface ou comme un implant.

3. Système orthopédique selon la revendication 1 ou 2,
**caractérisé en ce que** l'électrode de stimulation (10) est couplée au dispositif de commande (50).

4. Système orthopédique selon l'une des revendications précédentes, **caractérisé en ce que** l'accumulateur d'énergie (31) est réalisé comme un accumulateur à ressort, un accumulateur de pression ou un accumulateur pour énergie électrique.

5. Système orthopédique selon l'une des revendications précédentes, **caractérisé en ce qu'**un entraînement (32) est associé à l'ensemble formant orthèse ou prothèse (20).

6. Système orthopédique selon l'une des revendications précédentes, **caractérisé en ce que** l'ensemble formant orthèse ou prothèse (20) est réalisé comme une orthèse ou une prothèse de l'extrémité inférieure.

7. Système orthopédique selon l'une des revendications précédentes, **caractérisé en ce que** le dispositif de résistance (30) est réalisé comme un frein mécanique, comme un amortisseur à fluide, comme un amortisseur magnétorhéologique ou comme un générateur.

8. Système orthopédique selon la revendication 1,
**caractérisé en ce que** le système est conçu de telle sorte que les muscles agissant de manière antagoniste l'un par rapport à l'autre sont activés avec un décalage de temps au moyen d'au moins deux électrodes de stimulation (10).

9. Système orthopédique selon la revendication 8,
**caractérisé en ce qu'**un entraînement (32) associé à l'ensemble formant orthèse ou prothèse (20) soutient un mouvement de pivotement du composant proximal (21) par rapport au composant distal (22).

10. Système orthopédique selon l'une des revendications 1 à 9,
**caractérisé en ce que** le système est conçu de telle sorte qu'une activation des muscles s'effectue en fonction d'une force exercée sur un membre ou un composant (21, 22, 23, 41, 42) de l'ensemble formant orthèse ou prothèse (20) ou d'un couple exercé sur un membre ou un composant (21, 22, 23, 41, 42) de l'ensemble formant orthèse ou prothèse (20).

11. Système orthopédique selon l'une des revendications 1 à 10,
**caractérisé en ce que** le système est conçu de telle sorte qu'une activation des muscles est effectuée en fonction d'une accélération exercée par un membre ou un composant (21, 22, 23, 41, 42) de l'ensemble formant orthèse ou prothèse (20) et/ou en fonction de la position d'un membre ou d'un composant (21, 22, 23, 41, 42) de l'ensemble formant orthèse ou prothèse (20) dans l'espace ou l'un par rapport à l'autre.

12. Système orthopédique selon l'une des revendications 1 à 11,
**caractérisé en ce que** le système est conçu de telle sorte qu'il s'effectue une activation des muscles de la hanche et/ou des muscles de la cuisse.
